# EUROPÄISCHE PATENTSCHRIFT

(11) **EP 1 299 366 B1**
(45) Veröffentlichungstag und Bekanntmachung des Hinweises auf die Patenterteilung: **03.05.2006**
(21) Anmeldenummer: 01915324.6
(22) Anmeldetag: 09.03.2001
(51) Int. Cl.: C07D 279/20, B01J 2/18, C09K 15/30

(54) **VERFAHREN ZUR HERSTELLUNG VON PHENOTHIAZIN-GRANULAT MIT VERBESSERTEN EIGENSCHAFTEN**
METHOD FOR PRODUCING A PHENOTHIAZINE GRANULATE WITH IMPROVED PROPERTIES
PROCEDE DE PRODUCTION DE GRANULES DE PHENOTHIAZINE A PROPRIETES AMELIOREES

(30) Priorität: 01.07.2000 DE 10032137
(43) Veröffentlichungstag der Anmeldung: 09.04.2003
(73) Patentinhaber: Allessa Chemie GmbH, 60386 Frankfurt am Main (DE)
(72) Erfinder: BEYER, Jürgen, 60594 Frankfurt am Main (DE); BREIER, Dietmar, 63579 Freigericht (DE); EFFENBERGER, Gunther, 61118 Bad Vilbel (DE); ROOS, Michael, 65529 Waldems-Steinfischbach (DE); RUPPERT, Jens, 64711 Erbach (DE); JUST, Olaf, 61352 Bad Homburg (DE); WEHLE, Detlef, 65611 Brechen (DE)
(86) Internationale Anmeldenummer: PCT/EP2001/002681
(87) Internationale Veröffentlichungsnummer: WO 2002/002543

(56) Entgegenhaltungen:
- DE-A1- 4 338 212
- US-A- 3 160 630

## Beschreibung

Die vorliegende Anmeldung betrifft ein Verfahren zur Herstellung von Phenothiazin-Granulat mit verbesserten Löslichkeits- und Handlingseigenschaften.

Phenothiazin (2,3,5,6-Dibenzo-1,4-triazin, CAS-Nr. 92-84-2) ist Ausgangsstoff für Thiazinfarbstoffe und Schwefelfarbstoffe, Zwischenprodukt für die Herstellung von Arzneimitteln, ferner wird es eingesetzt als Antioxydans für Schmieröle und Motorenöle, als Antihelmintika (Wurmmittel im Sektor Veterinärmedizin), als Mittel gegen Obst-, Gemüse-, Getreide- und Baumwollschädlinge und im mengenmäßig größtem Umfang als Polymerisationsinhibitor für ethylenisch ungesättigte Carbonsäuren (Ullmann, XX. Auflage, Bd. 18, S. 259 ff; Römpps Chemie-Lexikon, 8. Aufl., S. 3133).

Die Herstellung von Phenothiazin erfolgt im technischen Maßstab durch Reaktion von Diphenylamin und Schwefel in Gegenwart von Katalysatoren. Dabei gebildeter Schwefelwasserstoff wird mit Natronlauge zu Natriumhydrosulfid gebunden. Das entstandene rohe Phenothiazin wird anschließend durch geeignete Reinigungsmethoden, z.B. durch Destillation unter verminderten Druck oder Wasserdampfdestillation, gereinigt. Der Schmelzpunkt von reinem Phenothiazin beträgt 185,5-185,9°C, der Siedepunkt bei Normaldruck beträgt 371°C.

Je nach Anwendungszweck wird Phenothiazin nach Herstellung und Reinigung konfektioniert, d.h. in geeignete feste Formen gebracht. Für den Einsatz als Anthelmintika wird Phenothiazin beispielsweise mit einer Partikelgröße kleiner als 30 µm, bevorzugt kleiner 20 µm eingesetzt (AU-B-254 331). In dieser Patentschrift wird die Herstellung von Phenothiazin mit einer spezifischen Oberfläche von 25,000 cms²/gm beschrieben, indem rohes oder kommerzielles Phenothiazin verdampft wird und anschließend in einem Gasstrom durch intensives Mischen der Gasströme kondensiert wird, wobei Phenothiazin mit einer Reinheit von > 95 % und in Form kristalliner Partikel mit einer spezifischen Oberfläche von mindestens 25,000 cms²/gm anfallen soll. In AU-B-254 331 wird aufgrund der ökonomischen Vorteile ferner die Verwendung einer Wirbelschicht zur Herstellung der beschriebenen Phenothiazinpartikel als bevorzugte Methode genannt. Weiter wird dort ausgeführt, dass das Wirbelschichtbett aus porösen Aluminiumsilikaten oder porösen Formen von Alkali- und Erdalkalimetallcarbonaten oder anderen Salzen besteht, auf die Phenothiazin mit dem Wirbelschichtverfahren aufgebracht wird.

In der US-A-3,235,453 werden weitere Methoden zur Herstellung von Phenothiazinpartikeln beschrieben. Im einzelnen werden genannt, die Zerkleinerung von vorzerkleinertem Phenothiazin mit einer Hammermühle, die Verwendung von Mikro-Pulverisierern, Kugelmühlen, Luftstrahlmühlen oder durch Nassvermahlung.

Mit dem Ziel der Herstellung von Phenothiazin sehr kleiner Partikelgröße wird in US-A-3,235,453 die Herstellung einer verbesserten Mischung beschrieben, wobei Phenothiazin in einem Lösungsmittel gelöst wird, mit einem Feststoff in Kontakt gebracht wird und anschließend das Lösungsmittel entfernt wird.

Alle genannten Methoden haben zum Ziel, Phenothiazin mit sehr kleiner Partikelgröße (für eine Verwendung als Antihelmintika) herzustellen, da die Wirkung von Kontaktgiften besser ist, je geringer die Partikelgröße ist.

Für den Einsatz als Polymerisationsinhibitor für ethylenisch ungesättigte Carbonsäuren wird Phenothiazin in fester Form eingesetzt und beispielsweise im Herstellprozess bei der Destillation von Acrylsäure im industriellen Maßstab eingesetzt. Phenothiazin verbleibt dabei im wesentlichen im Rückstand der Destillation. Phenothiazin ist ein derart effektiver Inhibitor für Acrylsäure, dass sein Einsatz gewöhnlicherweise zu Problemen bei der Polymerisation von Acrylsäure, dem Haupteinsatzgebiet, führt. Aus diesem Grund und auf Grund der dunklen Farbe von Phenothiazin wird Acrylsäure meistens mit anderen Inhibitoren, z.B.

Hydrochinonmonomethylether, einer farblosen Verbindung, inhibiert (L.B. Levy, Inhibition of Acrylic Acid Polymerization by Phenothiazine and p-Methoxyphenol, Journal of Polymer Science, Polymer Chemistry Edition, Vol. 23, 1505-1515, 1985).

Aus Dosiergründen und Gründen einer vereinfachten Handhabbarkeit wäre für den Einsatz von Phenothiazin als Stabilisator bei der Destillation von ethylenisch ungesättigten Carbonsäuren, wie z.B. Acrylsäure, der Einsatz von Phenothiazinlösungen durchaus erwünscht, dem stehen jedoch die schlechte Löslichkeit von Phenothiazin in gängigen Lösungsmitteln entgegen (z.T. deutlich unter 10%), womit entsprechend große Lagervorrichtungen notwendig wären. Die Auswahl an Lösungsmitteln ist ferner beschränkt durch die Tatsache, dass sie vollständig inert gegenüber Acrylsäure sein müssten und ferner bei der Destillation nicht überdestillieren dürfen, da ansonsten die Reinheit der Acrylsäure nicht den Ansprüchen genügen würde (Acrylsäure wird im allgemeinen in Polymerisationsprozessen eingesetzt, die sehr empfindlich auf Verunreinigungen reagieren).

Bis auf wenige Ausnahmen, z.B. Einsatz einer ca. 6 %igen Lösung von Phenothiazin in Ethylacetat als Shortstop-Inhibitor für Acrylsäure (hierunter ist die sehr rasche Zudosierung von Phenothiazin als Inhibitor für die Polymerisation von ethylenisch ungesättigten Carbonsäuren zu verstehen, z.B. im Falle von beginnender Polymerisation von nicht additivierter Acrylsäure oder bei Überhitzung von Lagerbehältern und in Folge Polymerisation mit der Folge einer runaway-Reaktion) wird daher in der industriellen Herstellung von ethylenisch ungesättigten Carbonsäuren Phenothiazin in fester Form eingesetzt.

Eine gebräuchliche Form ist die Herstellung und Verwendung von Phenothiazin in Schuppenform, wobei flüssiges Phenothiazin, z.B. nach erfolgter destillativer Aufreinigung, auf eine gekühlte Walze aufgebracht wird und die entstehende Schicht festen Phenothiazins mit einer Abstreifersystem in Form von Schuppen von der Walze gebrochen wird. Die Dicke der Schuppen ist dabei in gewissen Grenzen steuerbar, im allgemeinen können auf diese Weise Schuppen oder Plättchen mit einer Dicke von ca. 0,2 bis 4 mm und einer Ausdehnung von 0,2 bis 20 mm in den beiden anderen Dimensionen hergestellt werden. Bei der Herstellung der Schuppen selbst oder bei der anschließenden Förderung im Produktionsbetrieb zu Lagereinrichtungen oder später in entsprechenden Transportbehältnissen zum Verbraucher entsteht daneben noch Feinstaub mit einer Partikelgröße < 300 µm in Anteilen von bis zu 5%, der durch klassische Verfahren (z.B. Absieben und Rückführung in den Herstellprozess für Phenothiazin) weitgehend entfernt werden muss. Die Notwendigkeit für einen niedrigen Feinstaubanteil liegt darin begründet, dass Phenothiazin-Feinstaub eine erhöhte Tendenz zur Bildung explosionsfähiger Gemische in Luft aufweist, was somit von Relevanz für die Sicherheitstechnik beim Umgang mit diesem Stoff ist.

Aus der Beschreibung des Herstellprozesses für festes Phenothiazin folgt, dass das entstandene Feststoffpartikelkonglomerat inhomogen ist, in der Hinsicht, dass die Partikel eine relativ große Variabilität der Partikelgrößenverteilung in den genannten Grenzen, die als solche nur beispielhaft zu verstehen sind, aufweisen. Zudem können während des Transports im Herstellbetrieb oder beim Transport zum Verbraucher durch die mangelnde Scherstabilität wiederum Feinanteile gebildet werden, die aufgrund der höheren Staubexplosionsklasse (leichtere Zündfähigkeit in Mischung mit Luft, d.h. Zündung bereits bei geringerer Zündenergie, die durch Zündfunken, aber auch durch statische Elektrizität oder Reibung, eingebracht werden kann) für Feinstaub und dem erhöhten Risiko der Inhalation beim Umgang mit Phenothiazin, erhöhte Sicherheits- und Arbeitsschutzvorkehrungen notwendig machen.

Weiterhin ist das Löseverhalten von Phenothiazin in ethylenisch ungesättigten Carbonsäuren naturgemäß von der Partikelgrößenverteilung abhängig. Aus dem oben geschilderten folgt, dass in Abhängigkeit von der Länge des Transportweges bzw. unterschiedlicher mechanischer Belastungen unterschiedliche Lösungsgeschwindigkeiten beim Anwender zu erwarten sind, was erhöhten Kontrollaufwand, das Einrechnen von zeitlichen Puffern im Betriebsablauf, z.B. beim Löseprozess, erforderlich macht und letztlich eine ungenügende Prozessstabilität bedeutet.

Ein verbessertes und auch besser reproduzierbares Löslichkeitsverhalten sollte durch Herstellung und Anwendung von Phenothiazin extrem kleiner Partikelgröße erzielbar sein, dem stehen jedoch die bereits genannten sicherheitstechnischen Probleme und entsprechend zusätzlich zu treffende Sicherheitsvorkehrungen in Bezug auf Staubexplosionsfähigkeit sowie Probleme bei der Arbeitshygiene entgegen, wie auch die Tatsache, dass Feststoffe mit sehr geringer Partikelgröße eine nur geringe Schüttdichte aufweisen, was negative Auswirkungen auf die Transportökonomie hat. Ferner wird bei der Verwendung von Phenothiazin in Schuppenform und Versand in Großgebinden, z.B. in sogenannten big bags mit Füllgewichten bis zu 1 to in der Praxis Verbackung des Materials beobachtet, welches dann zeit- und personalaufwendig durch Anwendung mechanischer Verfahren wie Rütteln, Herausbrechen, Zerkleinern mit Gestängen in eine schüttfähige und dosierbare Form gebracht werden muss.

Es bestand daher die Aufgabe, ein Herstellverfahren für Phenothiazin zu entwickeln, welches die genannten Nachteile nicht besitzt, sondern Phenothiazin mit engerer Partikelgrößenverteilung, geringerem Feinkornanteil, konstanten und verbesserten Lösungseigenschaften und hoher Schüttdichte sowie verbesserten Fördereigenschaften gegenüber den Herstellverfahren des Standes der Technik liefert und zudem ökonomisch in der Herstellung ist.

Es wurde nun überraschend gefunden, dass die gestellte Aufgabe durch ein Verfahren zur Herstellung von Phenothiazin-Granulat mit enger Partikelgrößenverteilung gelöst wird, wobei Phenothiazin mit einer Reinheit von mindestens 98% in flüssiger Form durch eine mit Bohrungen versehene Einrichtung gepresst wird und in die Flüssigkeit auf geeignetem Wege eine Schwingung produktspezifischer Frequenz eingebracht wird, die die Bildung gleichförmiger Tropfen unterstützt. Das aus den Bohrungen austretende Phenothiazin tritt in ein Kühlmedium mit einer Temperatur von - 40°C bis + 100°C ein, wobei die erzeugten Phenothiazin-Flüssigkeitstropfen bis auf eine Temperatur unterhalb der Schmelztemperatur gebracht werden und diese gegebenenfalls in einer Nachkühlzone weiter verfestigt werden.

Der Partikeldurchmesser kann durch verschiedene Parameter gesteuert werden. Ein wesentlicher Parameter ist der Durchmesser der Bohrungen der Lochplatte. Erfindungsgemäß eignet sich zum Durchpressen des flüssigen Phenothiazins eine Düsenplatte mit Bohrungen mit einem Durchmesser im Bereich von 0,2 bis 1,5 mm, vorzugsweise mit einem Durchmesser im Bereich von 0,3 bis 0,9 mm, insbesondere mit einem Durchmesser im Bereich von 0,4 mm bis 0,6 mm.

Für die Herstellung des beschriebenen Phenothiazin-Granulates können auch Granulierapparate, wie sie z.B. zur Herstellung von Polyethylenwachsen, oxidiertem Polyethylen, Harzen mit geringem Molekulargewicht, ataktischem Polypropylen, Fetten oder Alkoholen oder Wachsmischungen eingesetzt werden, verwendet werden.

In genannten Granulierapparaten wird das zu granulierende oder zu pelletierende Phenothiazin in flüssiger Form durch eine Lochplatte gepresst, wobei auf das Phenothiazin eine Frequenz aufgebracht wird.
Üblicherweise liegt die aufzubringende Resonanzfrequenz im Bereich von 100 bis 10 000 Hz, vorzugsweise im Bereich von 200 bis 5000 Hz. Die optimale Frequenz zur Erzielung eines gleichmäßigen Tropfenabrisses kann von einem Fachmann durch Optimierungsversuche auf einfachem Wege ermittelt werden.

Die so gebildeten Flüssigkeitströpfchen werden in einem gekühlten Gasstrom (Kühlmedium) zu kugelförmigen bis ellipsoiden Feststoffpartikeln verfestigt. Nach der Verfestigung, die durch ganz oder teilweise Kristallisation begleitet sein kann, die zunächst im Außenbereich der Flüssigkeitströpfchen stattfindet, wird eine durchgehende Verfestigung oder Kristallisation im allgemeinen durch eine Nachkühlzone bewirkt.

Die Oberflächenstruktur wie auch die Porosität der Feststoffpartikel wird darüber hinaus noch durch andere Parameter beeinflusst, wie z.B. die Geschwindigkeit des entgegenströmenden Kühlmediums und die Temperatur des Kühlmediums.

Als Kühlmedium eignen sich Luft, Stickstoff oder Inertgase mit einer Temperatur im Bereich von -40 bis + 100°C, vorzugsweise mit einer Temperatur im Bereich von +20 bis +100°C.

Die Geschwindigkeit mit der das Kühlmedium den Phenothiazintropfen entgegenströmt liegt üblicherweise im Bereich von 0,1 bis 10 m/s vorzugsweise im Bereich von 0,5 bis 5 m/s.

Das erfindungsgemäße Verfahren erlaubt es ein Granulat mit einer Korngrößenverteilung im Bereich von 300-3000 µm, insbesondere mit einer Korngrößenverteilung im Bereich von 500 µm bis 2000 µm herzustellen. Der Volumenanteil der Partikel dieser Korngrößenverteilung am Gesamtvolumen liegt erfindungsgemäß bei mindestens 90 %, insbesondere bei ≥ 95 %.

Der Feinkornanteil, d.h. Partikel mit einer Größe < 300 µm liegt bei weniger als 3% Gewichtsanteil an der Gesamtmasse, im allgemeinen sogar bei weniger als 2% Gewichtsanteil an der Gesamtmasse an Granulat. Die gebildeten Feinkorn- sowie auch gegebenenfalls entstandene Grobkomanteile können durch einfache, dem Fachmann bekannte Methoden, z.B. durch Siebverfahren, abgetrennt werden.

Das mit dem erfindungsgemäßen Verfahren hergestellte Phenothiazin-Granulat verfügt über einen geringeren Feinkornanteil und über wesentlich verbesserte Löslichkeitseigenschaften als mit den bekannten Verfahren hergestellte Phenothiazin- Schuppen oder auch Pelletware.
Ferner konnte gezeigt werden, dass das erfindungsgemäß hergestellte Granulat eine bessere Scherstabilität besitzt, d.h. unter mechanischer Beanspruchung geringeren Abrieb zeigt als die vorstehend genannten bekannten Produkte.
So wurde überraschenderweise gefunden, dass bei Verwendung von gekühlter Luft oder gekühltem Inertgas im Temperaturbereich von -10 bis 20°C im Gegensatz zu verdampfenden Stickstoff als Kühlmedium bei der erfindungsgemäßen Herstellung ein Granulat mit höherer Schüttdichte und nochmals verbesserter Scherbeständigkeit, d.h. besserem Abriebverhalten erhalten werden konnte.

Die Schüttdichten der mit dem erfindungsgemäßen Verfahren erhaltenen Granulate liegen vorzugsweise im Bereich von 720 bis 780 kg/m³.

Die erfindungsgemäß hergestellten Granulate weisen ferner eine deutlich engere Partikelgrößenverteilung. Der Effekt des Abriebs durch eine Scherbeanspruchung ist bei den erfindungsgemäßen Phenothiazin-Granulaten wesentlich geringer als bei den mit dem bekannten Verfahren hergestellten Phenothiazinschuppen oder Pellets (siehe Beispiel 3).

Eine Beeinflussung des Löslichkeitsverhaltens des hergestellten Phenothiazin-Granulates kann beispielsweise durch die Variation der Temperatur des verwendeten Kühlmediums erreicht werden. So kann die Löslichkeit von Phenothiazingranulat in Acrylsäure deutlich verbessert werden, wenn das Kühlmedium bei der Herstellung, d.h. beim Kontakt bzw. beim Auftreffen auf das flüssige Phenothiazin, eine Temperatur im Bereich von -10 bis +80°C, vorzugsweise 0 bis +60°C aufweist. Bei der Verwendung von verdampfenden Stickstoff als Kühlmedium wird eine geringere, im Vergleich zu Phenothiazin-Schuppen aber trotzdem noch erhöhte, Löslichkeit erreicht (siehe Beispiel 2).

Die Lösegeschwindigkeit bis zum Erreichen einer Konzentration von 1,5 % in Acrylsäure liegt bei Raumtemperatur bei einer Korngrößenfraktion von 1000 bis 1400 µm des erfindungsgemäßen Granulates im Bereich von 5 bis 14 Minuten, insbesondere im Bereich von 7 bis 10 Minuten.

Die erfindungsgemäß herstellten Phenothiazin-Granulate eignen sich insbesondere im Hinblick auf ihre enge Partikelgrößenverteilung als Zusätze in Ölen und Schmiermitteln, als Polymerisationsinhibitor oder Stabilisator oder als Schädlingsbekämpfungsmittel in der Agrarwirtschaft.

### Beispiele:

### Methode zur Ermittlung des Löslichkeitsverhaltens von festem Phenothiazin unterschiedlicher Form und Partikelgrößenverteilung

Das Löslichkeitsverhalten wurde in Vergleichsversuchen bestimmt, indem 2 bis 3 Gew.-% Phenothiazin bezogen auf die Gesamtmasse bei Raumtemperatur zu kommerziell erhältlicher Acrylsäure (Aldrich, stabilisiert mit Hydrochinonmonomethylether) gegeben wurde. Die maximale Löslichkeit von Phenothiazin in Acrylsäure bei Raumtemperatur liegt bei ca. 2,8 % (m/m). Anschließend wurde in Zeitabständen von 1 bis 5 min entweder a.) eine Probe der Dispersion entnommen, filtriert und der Gehalt an Phenothiazin durch UV-Spektroskopie bestimmt oder b.) der Gehalt an Phenothiazin direkt mittels einer NIR-Sonde , die in die Dispersion von Phenothiazin in Acrylsäure eintauchte, bestimmt (NIRVIS-Universalspektrometer der Fa. Büchi mit Transmissionssonde mit 1,5 mm Spaltweite. Zur Verhinderung von Störungen durch Feststoffpartikel im Messspalt wurde dieser durch ein Metallsieb mit 0,18 mm Maschenweite verschlossen).

### Beispiel 1

### Bestimmung des Löslichkeitsverhalten von Phenothiazin-Pellets, Schuppen und Granulat

Nach der Methode 1b wurde das Löslichkeitsverhalten von Phenothiazin-Pellets (Halbkugeln bzw. Halbellipsoide mit Basisdurchmesser von 4-6 mm und Höhe von ca. 2-3 mm), Schuppen (Beschreibung siehe Text oben) und zwei auf verschiedene Weise hergestellten Granulaten (Granulat 1, Kühlmedium flüssiger bzw. verdampfender Stickstoff; Granulat 2, Kühlmedium Luft oder Inertgas (-10 bis +20°C)), verglichen. Hierzu wurden jeweils 66 g Acrylsäure mit 1,33 g der jeweiligen Probe versetzt und in Intervallen von einer Minute vermessen (Grafik 1).

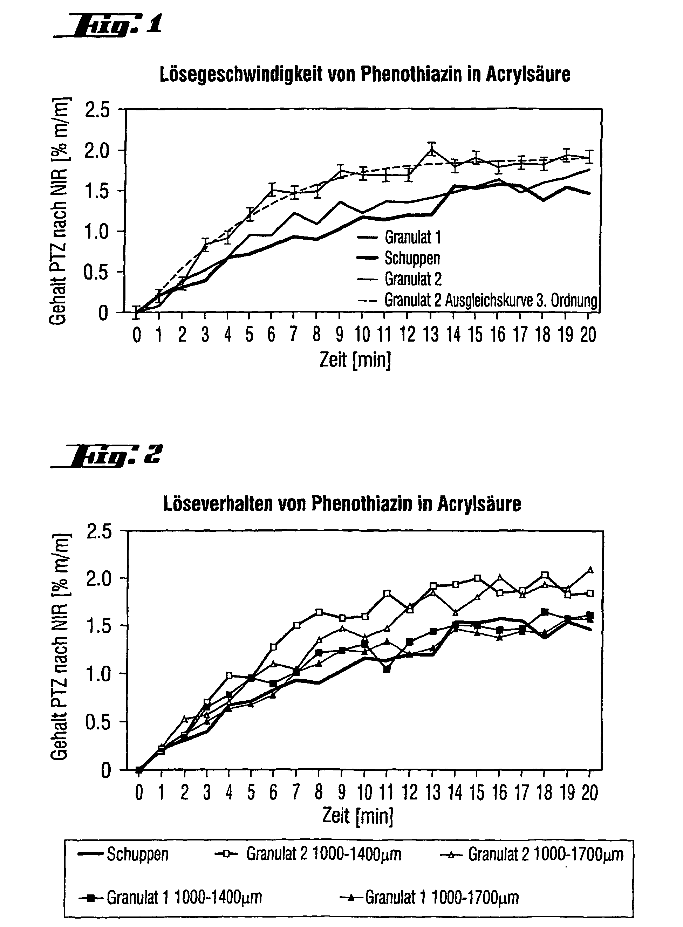

Aus den Löslichkeitskurven in Fig. 1 geht hervor, dass Granulat 2 (Kühlmedium Luft, Temperatur ca. 20°C) deutlich schneller in Lösung geht als Schuppen oder Granulat 1 (Kühlmedium verdampfender Stickstoff). [aus Gründen der Übersichtlichkeit wurden nur bei Granulat 2 Fehlerbalken mit eingezeichnet].

### Beispiel 2:

### Vergleich des Löslichkeitsverhaltens von Granulaten verschiedener Siebfraktionen und unterschiedlicher Herstellweise

Um auszuschließen, dass die beobachteten Unterschiede zwischen den durch Verwendung von Kühlmedium nahe Raumtemperatur hergestelltem Granulat und dem durch Verwendung von verdampfenden Stickstoff als Kühlmedium hergestelltem Granulat auf Unterschiede in den Partikelgrößenverteilungen zurückgeführt werden konnten, wurden von Granulat 1 und Granulat 2 zwei unterschiedliche Siebfraktionen (1000-1400 µm und 1000-1700 µm) hergestellt und die Löslichkeitseigenschaften dieser 4 Proben unter Anwendung von Methode 1 b ermittelt.

Die erhaltenen Ergebnisse sind in Fig. 2 dargestellt:
Man erkennt deutlich, dass Granulat 2 bei beiden Siebfraktionen, vor allem aber auch bei der Siebfraktion mit der Partikelgrößenverteilung im Bereich von 1000-1400 µm im Vergleich zu Granulat 1 deutlich schneller in Lösung geht. Man liest aus der Grafik ab, dass die Lösegeschwindigkeit bei der Korngrößenfraktion 1000-1400 µm bis zum Erreichen einer Konzentration von 1,5% mit ca. 7 min bei Granulat 2 gegenüber ca. 14 min bei Granulat 1 nahezu doppelt so schnell verläuft, was in der Praxis einen deutlichen Anwendungsvorteil darstellt.

### Beispiel 3

### Vergleichende Untersuchung des Abriebverhaltens von verschiedenen Phenothiazin-Partikeln

Als Maß für die Scherstabilität von verschiedenen Phenothiazin-Mustem und zur Simulation des Abriebverhaltens unter Transportbedingungen wurden Proben in einer Scherzelle eines Rotationsschergefäßes für einen Zeitraum von 30 min bei einer Normalspannung von 15 kPA einer Scherbeanspruchung unterworfen. Der Vergleich der Partikelgrößenverteilungen vor und nach der Messung lässt Aussagen über das Abriebverhalten der Partikel zu.

Die Partikelgrößenverteilungen sind in nachfolgenden Darstellungen dargestellt. Gezeigt wird die Volumen-Verteilungssummer auf der Ordinate und die Partikelgröße auf der Abszisse (logarithmischer Maßstab).

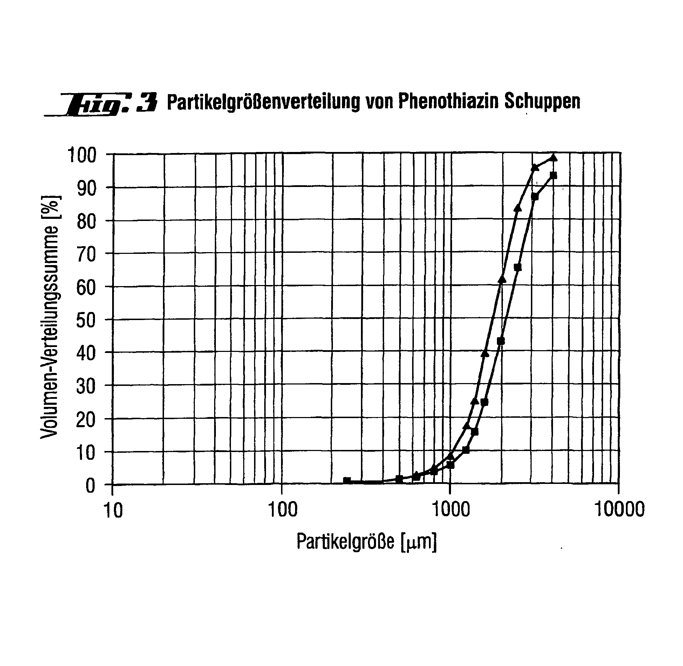

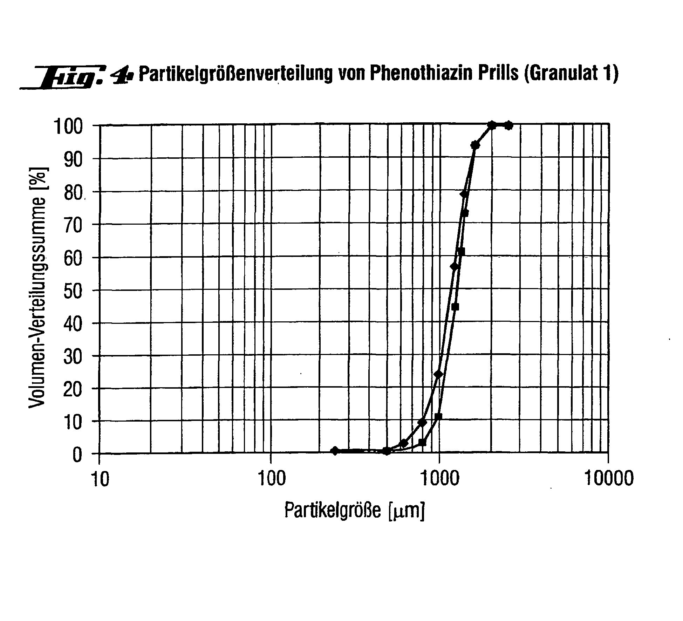

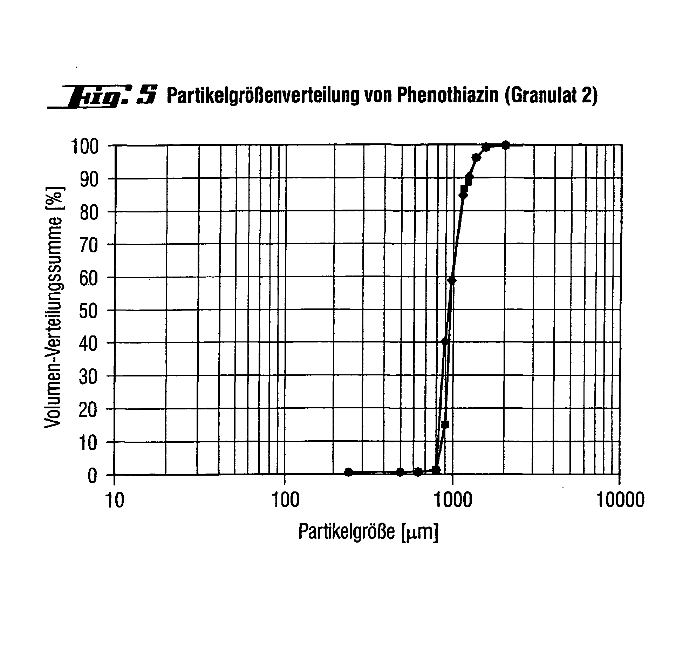

In der Fig. 3 (Phenothiazin Schuppen) stellen die ausgefüllten Kästchen den Volumenanteil der Partikel bis zu den angegebenen Partikelgrößen dar, wobei es sich um eine kumulative Darstellung handelt. Nach angegebener Scherbeanspruchung wurde die Partikelgrößenverteilung emeut bestimmt. Die im Mittel durchweg kleineren Partikel ist durch Verschiebung des Kurvenzuges nach links zu kleineren Partikelgrößen ersichtlich. Aus der Grafik geht auch die breite Partikelgrößenverteilung hervor, die sich von Partikeln < 200 µm bis hin zu Partikeln mit > 4000 µm (im ungescherten Zustand) erstreckt. Im Vergleich dazu weisen die Granulate 1 und 2 (Fig. 4 und Fig. 5) eine deutlich engere Partikelgrößenverteilung auf. Im Fall von Granulat 1 werden durch Scherbeanspruchung ebenfalls feinere Partikel gebildet, der Effekt ist jedoch bereits deutlich schwächer ausgeprägt als bei den Schuppen (geringere "Hysterese"). Bei Granulat 2 ist der Effekt nochmals schwächer ausgeprägt: hier ist nahezu kein Einfluss der Scherung auf die Partikelgrößenverteilung und damit auf den Abrieb zu beobachten.

### Beispiel 4

### Vergleich der Schüttdichten von Granulat 1 und 2

An je einer Proben von Granulat 1 und Granulat 2, die durch die nachfolgend aufgeführten Partikelgrößenverteilungen charakterisiert sind, wurden die Schüttdichten ermittelt, die im Falle von Granulat 2 mit 760 kg/m³ signifikant höher lagen als bei Granulat 1 mit 727 kg/m³:

| **Korngröße** | Granulat 1 | Granulat 2 |
|---|---|---|
| **3150-4000 µm** | 0,1 | < 0,1 |
| **2000-3150 µm** | 0,6 | 0,1 |
| **1000-2000 µm** | 68,8 | 33,6 |
| **500-1000 µm** | 28,1 | 65,4 |
| **250-500 µm** | 2,1 | 0,9 |
| **<250 µm** | 0,3 | <0,1 |
| **<100 µm** | 0,1 | < 0,1 |
| **<75 µm** | 0,1 | < 0,1 |
| | | |
| Schüttdichte | 727 kg/m³ | 760 kg/m³ |

## Patentansprüche

1. Verfahren zur Herstellung von Phenothiazin-Granulat mit einer Partikelgrößenverteilung im Bereich von 300 bis 3000 um, deren Volumenteil am Gesamtvolumen mindestens 90 % beträgt, wobei Phenothiazin mit einer Reinheit von mindestens 98 % in flüssiger Form durch eine mit Bohrungen versehene Einrichtung gepresst und auf das flüssige Phenothiazin eine Frequenz im Bereich von 100 bis 10 000 Hz aufgebracht wird und das durch die Bohrungen austretende flüssige Phenothiazin in ein Kühlmedium mit einer Temperatur im Bereich von 40 bis 100°C eintritt, wobei das Kühlmedium den aus den Bohrungen austretenden Phenothiazintropfen entgegenströmt, und als Kühlmedium gekühlte Luft oder gekühltes Inertgas verwendet wird, so dass die so erzeugten Phenothiazin-Flüssigkeitstropfen bis auf eine Temperatur unterhalb der Schmelztemperatur gebracht werden und diese gegebenenfalls in einer Nachkühlzone weiter verfestigt werden.

2. Verfahren nach Anspruch 1, **dadurch gekennzeichnet, dass** die mit Bohrungen versehene Einrichtung eine Düsenplatte ist.

3. Verfahren nach mindestens einem der vorhergehenden Ansprüche, **dadurch gekennzeichnet, dass** als Kühlmedium Stickstoff oder Luft verwendet wird.

4. Verfahren nach mindestens einem der vorhergehenden Ansprüche, **dadurch gekennzeichnet, dass** der Feinkornanteil mit Teilchen < 300 µm weniger als 3 %, beträgt.

5. Verfahren nach mindestens einem der vorhergehenden Ansprüche, **dadurch gekennzeichnet, dass** gegebenenfalls gebildete Feinkomanteile oder Grobkomanteile durch geeignete Methoden entfernt werden.

6. Verfahren nach mindestens einem der vorhergehenden Ansprüche, **dadurch gekennzeichnet, dass** die Schüttdichte des erhaltenen Phenothiazin-Granulats im Bereich von 720 bis 780 kg/m³ liegt.

7. Verfahren nach mindestens einem der vorhergehenden Ansprüche, **dadurch gekennzeichnet, dass** die Lösegeschwindigkeit in Acrylsäure von Phenothiazin-Granulat mit einer Korngrößenfraktion im Bereich von 1000 bis 1400 µm bis zum Erreichen von einer Konzentration von 1,5 % bei 5 bis 14 Minuten liegt.

8. Phenothiazin-Granulate enger Partikelgrößenverteilung erhältlich gemäß eines Verfahrens einer oder mehrerer der Ansprüche 1 bis 7.

9. Verwendung von Phenothiazin-Granulaten enger Partikelgrößenverteilung gemäß Anspruch 8 als Zusätze in Ölen und Schmiermitteln, als Polymerisationsinhibitor oder Stabilisator oder als Schädlingsbekämpfungsmittel in der Agrarwirtschaft.

## Claims

1. A process for the preparation of phenothiazine granules having a particle size distribution, in the range of from 300 to 3 000 µm, the volume fraction thereof being at least 90%, based on the total volume, phenothiazine having a purity of at least 98% in liquid form being forced through a means provided with holes and a frequency in the range from 100 to 10 000 Hz being applied to the liquid phenothiazine and the liquid phenothiazine emerging through the holes entering a cooling medium having a temperature in the range of from -40 to +100°C, the cooling medium flowing countercurrent to the phenothiazine drops emerging from the holes, and cooled air or cooled inert gas being used as the cooling medium, so that the liquid phenothiazine drops thus produced are brought to a temperature below the melting point and said drops are, if required, further solidified in a downstream cooling zone.

2. The process as claimed in claim 1, wherein the means provided with holes is a die plate.

3. The process as claimed in at least one of the preceding claims, wherein the cooling medium used is nitrogen or air.

4. The process as claimed in at least one of the preceding claims, wherein the fine particle fraction having particles of < 300 µm is less than 3%.

5. The process as claimed in at least one of the preceding claims, wherein any fine particle fractions or coarse particle fractions formed are removed by suitable methods.

6. The process as claimed in at least one of the preceding claims, wherein the bulk density of the phenothiazine granules obtained is in the range of from 720 to 780 kg/m³.

7. The process as claimed in at least one of the preceding claims, wherein the dissolution rate, in acrylic acid, of phenothiazine granules having a particle size fraction in the range of from 1 000 to 1 400 µm up to reaching a concentration of 1.5% is from 5 to 14 minutes.

8. Phenothiazine granules having a narrow particle size distribution, obtainable by a process of one or more of claims 1 to 7.

9. The use of phenothiazine granules having a narrow particle size distribution as claimed in claim 8 as additives in oils and lubricants, as a polymerization inhibitor or stabilizer or as pesticides in agriculture.

## Revendications

1. Procédé de préparation de granulé de phénothiazine avec une répartition granulométrique dans la gamme de 300 à 3000 µm, dont la proportion en volume s'élève à au moins 90% du volume total, dans lequel on presse de la phénothiazine avec une pureté d'au moins 98 % sous forme liquide à travers un dispositif muni de trous et on applique une fréquence dans la gamme de 100 à 10 000 Hz à la phénothiazine liquide et la phénothiazine sortant à travers les trous entre dans un milieu de refroidissement avec une température dans la gamme de 40 à 100 °C, le milieu de refroidissement circulant à contre-courant des gouttes de phénothiazine sortant des trous, et on utilise comme milieu de refroidissement de l'air refroidi ou un gaz inerte refroidi, de sorte que les gouttes de liquide de phénothiazine ainsi produites sont amenées à une température inférieure à la température de fusion et celles-ci continuent à se solidifier éventuellement dans une zone dans une zone de refroidissement en aval.

2. Procédé selon la revendication 1, **caractérisé en ce que** le dispositif muni de trous est une plaque de buses.

3. Procédé selon au moins l'une des revendications précédentes, **caractérisé en ce que** l'on utilise comme milieu de refroidissement l'azote ou l'air.

4. Procédé selon au moins l'une des revendications précédentes, **caractérisé en ce que** la proportion de particules fines avec des particules < 300 µm est inférieure à 3 %.

5. Procédé selon au moins l'une des revendications précédentes, **caractérisé en ce que** les parties de fines particules ou les parties de grosses particules éventuellement formées sont éliminées par des procédés appropriés.

6. Procédé selon au moins l'une des revendications précédentes, **caractérisé en ce que** la masse volumique non tassée du granulé de phénothiazine obtenu se situe dans la gamme de 720 à 780 kg/m³.

7. Procédé selon au moins l'une des revendications précédentes, **caractérisé en ce que** la vitesse de dissolution dans l'acide acrylique du granulé de phénothiazine avec une fraction granulométrique dans la gamme de 1000 à 1400 µm est entre 5 et 14 minutes pour atteindre une concentration de 1,5 %.

8. Granulé de phénothiazine de répartition granulométrique étroite pouvant être obtenu selon un procédé d'une ou de plusieurs des revendications 1 à 7.

9. Utilisation de granulés de phénothiazine de répartition granulométrique étroite selon la revendication 8 comme additifs dans des huiles et des lubrifiants, comme inhibiteur de polymérisation ou stabilisateur ou comme agent de lutte contre les nuisibles dans l'économie agricole.
